# EUROPEAN PATENT SPECIFICATION

(11) **EP 1 534 246 B1**
(45) Date of publication and mention of the grant of the patent: **26.11.2008**
(21) Application number: 03787944.2
(22) Date of filing: 18.08.2003
(51) Int. Cl.: A61K 9/22, A61K 31/545, A61K 31/546

(54) **SUSTAINED RELEASE PHARMACEUTICAL COMPOSITION OF A CEPHALOSPORIN ANTIBIOTIC**
PHARMAZEUTISCHE ZUSAMMENSETZUNG MIT VERZÖGERTE FREISETZUNG ENTHALTEND EINEN CEPHALOSPORIN-ANTIBIOTIKUM
COMPOSITION PHARMACEUTIQUE DE CEPHALOSPORINE A LIBERATION PROLONGEE

(30) Priority: 16.08.2002 IN MA06012002; 19.08.2002 US 222930; 19.08.2002 WO PCT/IB02/03320
(43) Date of publication of application: 01.06.2005
(73) Proprietor: Orchid Chemicals and Pharmaceuticals Ltd, Nungambakkam, 600 034 Chennai (IN)
(72) Inventor: KSHIRSAGAR, R.S. Sree Lakshmi Ap., Flat no. 1/11, Shastrinagar Adyar 600020 Chennai (IN); BOLDHANE, S.P., Ramnagar Velachery 600042 Chennai (IN); JINDAL, Kour, Chand, Panchkula 134113 Haryana (IN)
(74) Representative: Wichmann, Hendrik
(86) International application number: PCT/IB2003/003340
(87) International publication number: WO 2004/016251

(56) References cited:
- WO-A-02/41876
- WO-A-98/46213
- WO-A-02/067943

## Description

### Field of the Invention

This invention relates to a sustained release pharmaceutical composition comprising at least a cephalosporin antibiotic, a mixture of polymers, and other pharmaceutically acceptable excipients as defined in the claims. The polymers are selected from mixture comprising of galactomannans and neutral swellable polymers, which release the active ingredient in a predetermined manner, said galactomannans being xanthan gum and neutral swellable polymer being poly (ethyl acrylate: methyl methacrylate) 2:1.

### Background of Invention

While many compounds are known to be useful as pharmacologically active substances, some of them have relatively short biological half life and needs to be administered several times a day in order to achieve desired therapeutic effects. Especially, the drugs used in treatment of microbial infections are required to be given more than once during a dosage regimen.

In an anti-microbial therapy the main requirement is to maximize the blood concentration, preferably several folds higher than the minimum inhibitory concentration (MIC) for the active agent, yet to minimize both the risk of toxicity to the patient and of promoting microbial resistance. Although oral administration will be the preferred route, in the case of antibiotics this route is frequently unattractive because of their low or variable oral bioavailability. In addition extremely high plasma concentrations of antibiotics are frequently required to achieve the MIC values towards certain gram negative bacteria (antibiotics and chemotherapy; anti-infective agents and their use in therapy 7th edition Ed. by O'grady F, Finch RG, Lambert HP; Greenwood D; Churchill Livingstone 1997).

Sustained release preparation of drugs are advantageous as the administration frequency can be reduced by maintaining a constant plasma concentration of drug over an extended period of time to ensure sustained effect of active ingredient well above the MIC levels. In addition, these preparations are also expected to decrease side effects by suppressing the rapid rise in the blood levels of the drug.

This has been primarily achieved by development of a novel drug delivery system utilizing diverse techniques and principles. Amongst these, known in the art is one such delivery system, which employs hydrophilic polymers to sustained or modified release pharmaceutical composition. For modified release solid dosage forms comprising a drug dispersed uniformly in mixture of polymers, release of the drug is controlled primarily by diffusion of the drug, or by surface erosion of the hydrophilic polymers into the surrounding medium or by a combination of both processes. Control of the rate of release can produce constant blood levels of the active ingredient that may result in reducing the frequency of administration, thereby improving patient compliance to the dosage regimen.

The relevant prior art methods, which teach adaptation of diverse delivery system for sustained release of the active ingredient, are as follows:
United States Patent No. 6,120,803 discloses an active agent dosage form, which is adapted for retention in the stomach and useful for the prolonged delivery of an active agent to a fluid environment. The active agent dosage form is a polymer composition that swells upon contact with the fluid of the stomach. A portion of the polymer composition is surrounded by a band of insoluble material that prevents the covered portion of the polymer composition from swelling and provides a segment of the dosage form that is of sufficient rigidity to withstand the contraction of the stomach and delay expulsion of the dosage form from the stomach until substantially all the active agent has been dispensed.
United States Patent No. 5,128,142 discloses a controlled release formulation comprising an absorbate of a mixture of pharmaceutically active ingredients and an inactive substance absorbed on a cross-linked polymer. The inactive substance is selected to modify the dissolution of active ingredients from the cross linked polymer *in vivo.* The inactive substance is preferably present in the absorbate in an amount of 0.5 - 3 parts by weight relative to 1 part by weight of the active ingredients.
United States Patent No. 4,968,508 discloses a sustained release matrix tablet comprising from about 0.1 % to about 90% by weight of Cefaclor, about 5% of about 29% by weight by hydrophilic polymer and about 0.5% to about 25% by weight of an acrylic polymer which dissolve at a pH in the range of about 5.0 to about 7.4, the total weight of polymers being less than 30% by weight of the formulation. Although a specific Cefaclor formulation is claimed the text suggests that the matrix formulation is suitable for weakly basic drugs and particularly suitable for Cephalexin and Cefaclor.
International Publication number WO 02/41876 discloses a pharmaceutical composition in the form of a tablet for controlled release of an active ingredient comprising a cephalosporin antibiotic such as Cephalexin, Cefaclor or their pharmaceutically acceptable hydrates, salts or esters as active ingredients, and a mixture of hydrophilic polymers selected from the group consisting of at least one sodium alginate and at least one xanthan gum as controlled release matrix and optionally probenecid as an antibiotic adjuvant as either immediate release or controlled release part. The composition may contain one or more of a water soluble and/or water dispersible diluent. The quantity of the hydrophilic polymers matrix still provides the desired once a day profile.
International Publication number WO 02/36126 discloses a fast disintegrating controlled release oral composition comprising a core material containing Cefuroxime axetil present as controlled release form, the Cefuroxime axetil being provided with an outer coating of a copolymer selected from aqueous dispersions of enteric methacrylic acid and methacrylic acid esters anionic copolymers having carboxyl group as the functional group or mixture thereof and an inner coating of a sustained release copolymer selected from aqueous dispersions of acrylate or methacrylate pH independent copolymers having quaternary ammonium group as a functional group or mixture thereof. Additionally the coating composition may contain plasticizers. The composition is suitable for once daily administration.
United States Patent No. 4,250,166 discloses a long acting Cephalexin preparation comprising of normal quick releasing Cephalexin and particulate Cephalexin coated with a copolymer of methacrylates and methacrylic acid which dissolves at pH from 5.5 to 6.5 and the potency ratio of the normal Cephalexin to coated Cephalexin is between 40:60 and 25:75.
United States Patent No. 6,399,086 discloses a controlled release cephalosporin antibiotic agent preferably Amoxicillin trihydrate in a hydrophilic and / or hydrophobic polymer matrix such that at least 50% but not more than 67.61 ± 5.78 % of the active agent is released within 3 to 4 hrs from oral administration and remainder is released at a controlled rate from the said composition. The composition teaches the use of commonly used hydrophilic polymers such as hydrophilic cellulose derivatives, hydrophilic methacrylic acid derivatives, chitosan, and alginates. Preferably, they have used hydrophilic cellulose derivative such as methylcellulose, hydroxypropyl methycellulose, hydroxyethyl cellulose. The hydrophobic polymers used in the invention are acrylamides and polyamido derivatives and hydrophobic methacrylic acid derivatives. The preferred hydrophobic polymer is ethyl cellulose. However with this profile wherein 67.61 ± 5.78% of the drug is already out of the matrix, and considering a very short half-life of most cephalosporins such as Cephalexin and Cefprozil (55 min and 70 min) respectively the drug profile achieved cannot be suitable for once daily administration. Hence, it is necessary that the matrix formulation should release the drug over extended period of time.
The corresponding International Publication number WO 98/22091 discloses a controlled release oral drug delivery system comprising as active ingredient a beta-lactam antibiotic having a specific absorption site in a small intestine in a hydrophilic and / or hydrophobic polymer matrix such that at least 50% of the active agent is released within 3 to 4 hrs from oral administration and remainder is released at a controlled rate from the said composition.
United States Patent No. 6,083,532 discloses a sustained release tablet comprising a drug to be released at a controlled rate and a sustained release formulation comprising at least three different types of polymers including a pH dependent gelling polymer, a pH independent gelling polymer and an enteric polymer. The pH dependent gelling polymer comprises at least one of an alginate, a carboxyvinyl polymer, or a salt of a carboxymethyl cellulose. The pH independent gelling polymer comprises at least one of a HPMC, HPEC, a HPC, a HEC, a methylcellulose, a xanthan gum or a polyethylene oxide. The enteric polymer comprises at least one of a polyacrylate material, a cellulose acetate phthalate, a cellulose phthalate hydroxy propyl methyl ether, a polyvinyl acetate phthalate, a hydroxy propyl methyl cellulose acetate succinate, a cellulose acetate trimelliatate or a shellac.
United States Patent No. 5,948,440 discloses a controlled release tablet of an active ingredient comprising of Cefaclor, Cephalexin or their pharmaceutically acceptable hydrates, salts or esters as active ingredients and a mixture of hydrophilic polymers selected from the group consisting of at least one hydroxy propyl methylcellulose and at least one hydroxyl propyl cellulose. The composition optionally also contains one or more of a water soluble or water dispersible diluent, the quantities are such that the therapeutically effective active ingredient is released at a rate for twice daily administration of the pharmaceutical composition.
Japanese Patent JP 57165392A discloses a long acting Cephalexin tablet comprising Cephalexin mixed with ≥ 10% w/w oils and fats (e.g. higher fatty acid, higher alcohol, alcohol ester etc) and with a vehicle such as magnesium stearate and the mixture is pressed, formed into granules passing through a 20 mesh sieve, and subjected to the slug formed process to obtain a high quality long acting tablets. The rate of dissolution of Cephalexin can be controlled by selecting the kind of oils and fats and the number of the time of slug formation process.
International Publication number WO 00/15198 teaches controlled delivery pharmaceutical composition having temporal and spatial control, comprising a drug, a gas generating component a swelling agent, a viscolyzing agent and optionally a gel forming polymer. The viscolyzing agent initially and the gel forming polymer thereafter form a hydrated gel matrix which entraps the gas, causing the tablet to float so that it is retained in the stomach thereby providing spatial control and at the same time resulting in sustained release of the drug providing temporal control.

The combination of gas generating component, swelling agent and viscolyzing agent results in the controlled drug delivery systems. Thus all these components are essential for achieving the temporal and spatial control. A preferred once daily Ciprofloxacin formulation comprising 69.9% Ciprofloxacin base, 0.34% sodium alginate, 1.03% xanthan gum, 13.7% sodium bicarbonate, 12.1% cross linked polyvinyl pyrrolidone and optionally other excipients is disclosed.

Sustained release preparation of drugs are advantageous in the administration, frequency can be reduced by maintaining a constant plasma concentration of drug over an extended period of time to ensure sustained effect of active ingredient.

Since the antibiotics are high frequency / high dosing, extended release drug delivery systems have not been very successful in reducing the frequency. The present invention is based on the observation that the release of active ingredient from the delivery system is controlled by the specific polymers present in the matrix and in specific concentrations, thus allowing blood levels above MIC over extended period of time such that the frequency of the dosage form administration can be reduced to twice daily or once daily.

### Objective of the Invention

The main objective of the present invention is to provide sustained release pharmaceutical compositions, which have blood levels above MIC over extended period of time.

Another objective of the present invention is to provide sustained release pharmaceutical compositions suitable for twice daily or once daily dosage form.

Yet another objective of the present invention is to provide sustained release pharmaceutical compositions, which release the active ingredient in a predetermined manner.

Yet another objective of the present invention is to provide sustained release pharmaceutical compositions of a cephalosporin antibiotic.

### Summary of the Invention

Accordingly, the present invention relates to a sustained release pharmaceutical composition comprising a cephalosporin antibiotic, a mixture of galactomannan and neutral swellable polymer and other pharmaceutically acceptable excipients as defined in the claims. The polymer is selected in such a way to give sustained release of the active ingredient in a predetermined manner.

In a first aspect, the invention relates to sustained release pharmaceutical compositions comprising a cephalosporin antibiotic, a mixture of galactomannan and neutral swellable polymer, said galactomannan being xanthan gum and neutral swellable polymer being poly (ethyl acrylate: methyl methacrylate) 2:1, wherein the composition comprises from 30% to 90% by weight of a cephalosporin antibiotic, 1% to 30% by weight of said mixture from 0.1% to 15% by weight of xanthan gum and from 0.1% to 15% by weight of poly(ethyl acrylate:methyl methacrlate)2:1, as defined in the claims.

Preferably, the present invention relates to the sustained release pharmaceutical composition comprises 30 % to 90 % by weight of cephalosporin antibiotic, 1 % to 20 % by weight of mixture of polymers comprising of galactomannans in an amount from 0.1 % to 12 % by weight and neutral swellable polymer in an amount from 0.1 % to 12 % by weight of sustained release composition.

According to yet another embodiment of the present invention, the sustained release pharmaceutical composition may be prepared by wet granulation method, the said method comprising the steps of:
(i) mixing the active ingredient, excipients and galactomannans in a mixer,
(ii) granulating the mixture with poly (ethyl acrylate:methyl methacrylate) 2:1,
(iii) drying the granules by either tray drier or fluid bed drier,
(iv) milling the dried granules followed by blending the same with addition of dry binder(s) and lubricant(s),
(v) compressing the lubricated granules into tablets using a tablet press and, if desired, coating the tablets.

According to still another embodiment of the present invention, the sustained release pharmaceutical composition may be prepared by optionally compacting the mixture and sizing it by passing through a sieve after step (i) mentioned above, then followed by the rest of the steps.

A binder such as low viscosity hydroxypropyl methylcellulose (HPMC) can be added optionally during wet granulation.

The tablets may be optionally coated using conventional coating agents. The composition of the present invention may also be formulated as other sustained release oral dosage forms such as capsules, etc.

### Detailed Description of the Invention

In an embodiment of the present invention, cephalosporin antibiotic used as active ingredient include Cephalexin, Cefprozil, Cefditoren pivoxil, Cefadroxil, Cefpodoxime proxetil, Cefiuoxime axetil, Cefaclor, Cefamandole, Cefoxitin, Cephalothin, Cephapirin, Ceftizoxime, Cefonicid and their pharmaceutically acceptable hydrates, salts or esters.

In another embodiment of the present invention, the cephalosporin antibiotic may be present in an amount from about 30 to about 90% by weight of the sustained release composition. Further, the cephalosporin antibiotic may be present in the amount from 50 mg to 2000 mg.

Further, galactomannans selected from the group consisting of xanthan gum, guar gum, locuat bean gum and the like are contemplated.

In the present invention, the neutral swellable polymer used is Poly (ethyl acrylate: methyl methacrylate) 2:1.

The composition optionally comprises of one or more excipients such as water soluble or water dispersible diluents, binders, lubricant wherein the quantities of galactomannans and neutral swellable polymers, and water soluble / water dispersible diluents are such that the therapeutically effective active ingredients is released at a rate suitable for once or twice daily administration of the dosage form.

The composition may contain one or more pharmaceutically acceptable diluent(s) in amount from about 1% to about 50 % by weight of the total weight of composition.

These diluents may be water soluble or water dispersible. Examples of water soluble diluents that may be used in the present invention include lactose, mannitol, glucose, sorbitol, maltose, dextrates and, dextrins. Water dispersible diluents that may be used such as microcrystalline cellulose, pregelatinized starch, calcium carboxymethylcellulose, croscarmellose sodium and, sodium starch glycollate. In the preferred embodiment, the diluent is lactose in amount from 5 % to 20 % by weight of the sustained release composition.

The composition may also contain a tablet binder at a concentration in the range of 0.2 % to 12 % by weight of the total weight of composition. The binder may include, polyvinyl pyrrolidone, hydroxypropyl methylcellulose, hydroxypropyl cellulose, hydroxyethyl cellulose, gelatin, pregelatinized starch, sugar, and copolyvidonum (Plasdone S-630).

The composition may also contain a tablet lubricant at a concentration in the range of 0.2 % to 5 % by weight of the total weight of composition. The lubricant that may be used include, talc, stearic acid, magnesium stearate, colloidal silicon dioxide, calcium stearate, zinc stearate, and hydrogenated vegetable oil.

The composition of the present invention may also contain a glidant such as colloidal silicon dioxide.

Xanthan gum is a high molecular weight, anionic, natural heteropolysaccharide gum produced by aerobic fermentation with the organism *xanthomonas campestris.* It contains D-glucose, D-mannose, D-glucuronate in the molar ratio of 2.8: 2.0: 20 and is partially acetylated with about 4.7% acetyl. Xanthan gum also includes about 3.0% pyruvate, which is attached to a single unit D-glucopyromosyl side chain as a metal. It dissolves in hot or cold water and the viscosity of aqueous solutions of xanthan gum is only slightly affected by changes in the pH of solution between 1 and 11.

Xanthan gum has a branched or helical configuration, thus results in excellent water wicking properties. When xanthan gum comes into contact with an aqueous medium of gastrointestinal tract, it hydrates and swells to form a gel. It has good swelling action on contact with an aqueous medium and overcomes the problem encountered by other gums, which either do not hydrate rapidly enough or hydrate too rapidly. Xanthan gum alone when used as a matrix forming agent in sustained release tablets, releases the drug slightly faster in acidic media, due to more rapid initial surface erosion than at higher pH. After hydration of the gum the drug release is essentially pH independent.

During the course of our studies we found that when xanthan gum alone was used as matrix forming agent, the initial release of the cephalosporin was rapid, but the release retarded at later stage due to hydration of xanthan gum to form a gel.

| ***Ingredients*** | ***Weight (mg* / *tablet)*** | **% *wlw*** |
|---|---|---|
| Cephalexin | 795.32 | 75.74 |
| Lactose anhydrous | 139.18 | 13.26 |
| Xanthan gum | 105.0 | 10.0 |
| Magnesium stearate | 10.5 | 1.0 |

Dissolution in USP Apparatus Type I (900 ml 0.1N HCl, 100 RPM) followed by pH 6.8 phosphate buffer is shown below:

| ***Time (hour)*** | ***Percent Cephalexin Released*** |
|---|---|
| 1 | 40.0 |
| 2 | 45.3 |
| 3 | 50.2 |
| 4 | 56.4 |
| 5 | 60.2 |
| 6 | 62.1 |

Xanthan gum polymers that may be used in the present invention include Xantural ^{™} (Kelco), Rhodigel ^{™} available from (Rhodia, USA) and xanthan gum (Jungbunzlauer, Austria.)

Poly (ethyl acrylate: methyl methacrylate) 2:1 is a latex available only in aqueous dispersion either 30% or 40 % solids including neutral emulsifier. It has no functional group and is practically neutral. Therefore the films formed are insoluble in water or in aqueous medium over the entire pH range. The polymer swells in aqueous media and gives permeable membrane. The permeability is independent of pH.

We also found that when poly (ethyl acrylate : methyl methacrylate) 2:1 was used alone the release was retarded, however the tablet integrity was lost after 2 hours.

| ***Ingredients*** | ***Weight (mg* / *tablet)*** | **% *wlw*** |
|---|---|---|
| Cephalexin | 795.32 | 75.74 |
| Lactose anhydrous | 139.18 | 13.26 |
| Eudragit NE 30 D | 105.0 | 10.0 |
| Magnesium stearate | 10.5 | 1.0 |

Dissolution in USP Apparatus Type I (900 ml 0.1N HCl, 100 RPM) followed by pH 6.8 phosphate buffer is shown below:

| ***Time (hour)*** | ***Percent Cephalexin Released*** |
|---|---|
| 1 | 18.9 |
| 2 | 61.76 |
| 3 | 98.6 (Integrity lost) |

Poly (ethyl acrylate: methyl methacrylate) 2:1 is available under the brand name of Eudragit NE 30D from Rohm Pharma Company, Germany.

According to the present invention, when a pH independent neutral swellable polymer was mixed with xanthan gum along with active ingredient, the release from the composition was controlled initially and released uniformly over a period of time such that desired blood levels were achieved suitable for twice or once daily dosage form.

The combination of xanthan gum and poly (ethyl acrylate: methyl methacrylate) 2:1 is a unique combination suitable for sustained release of active ingredients, which are to be administered once daily. Both the polymers give a pH independent release profile.

The granulation of the blend of active ingredient, diluent, binder, lubricant and xanthan gum with poly (ethyl acrylate: methyl methacrylate) 2:1 aqueous dispersions, form thinner but more effective film layers, where drug particles and granulating excipients are partially impregnated and during compression they are embedded in a fine network of thin polymer layers. When the tablet comes in contact with aqueous media of the gastrointestinal tract, the thin film of poly (ethyl acrylate: methyl methacrylate) 2:1 controls the penetration of digestive fluids in to the composition and thus avoids initial erosion of xanthan gum, which is high at acidic pH. The poly (ethyl acrylate: methyl methacrylate) 2:1 slowly hydrates without disrupting the hydrophilic composition formed by the heteropolysaccharide. The insoluble poly (ethyl acrylate: methyl methacrylate) 2:1 forms a sponge like structure, which behaves as an inert matrix. Once the xanthan gum is completely hydrated, it forms a gel and then the release of active ingredient is governed by diffusion of dissolved drug through the pores, channels and capillaries of the insoluble polymer composition.

In the present invention it was observed that when these two polymers were mixed together in appropriate concentration to form a tablet, the release was controlled in such a manner that the dosage form is suitable for once daily administration.

We have found surprisingly that when we control the release of the active ingredients so as to achieve a 14 - 16 hours release profile, we were able to achieve the blood levels suitable for dosage forms for once or twice daily administration.

The present invention is illustrated by the following examples.

### Examples

### General procedure for the preparation of sustained release tablet:

Cephalosporin antibiotic, galactomannans, lactose were screened through 30 mesh sieve and granulated with aqueous dispersions of Eudragit NE 30D by using high shear Mixer granulator or Fluidized bed processor. Granules were dried in either Tray drier or Fluidized bed drier. The dried granules were milled, followed by blending with addition of dry binder such as low viscosity hydroxypropyl methylcellulose and lubricant such as magnesium stearate. The lubricated granules were then compressed to form tablets. These core tablets were optionally coated using conventional coating agents such as hydroxypropyl methylcellulose (HPMC), Opadry^{™}, and plasticizers such as polyethylene glycol, propylene glycol, etc.

The composition of the present invention may also be formulated as other sustained release oral dosage forms such as powders, pellets, minitablets, filled into capsules, etc.

### Dissolution method:

For all the examples containing Cephalexin, Cefprozil or Cefadroxil, wherever specific dissolution conditions are not mentioned, the tablets were tested for Cephalexin, Cefprozil or Cefadroxil in "USP Apparatus Type I (Basket method)" in 900 ml of 0.1N HCl for 1 hour, after which the dissolution media was changed to 900 ml of pH 6.8 phosphate buffer. The tablets were placed in 40-mesh basket and rotated at 100 RPM, and tested at different specified time points.

For all the examples containing Cefpodoxime proxetil or Cefuroxime axetil, wherever specific dissolution conditions are not mentioned, the tablets were tested for Cefpodoxime proxetil or Cefuroxime axetil in "USP Apparatus Type II (Paddle method)", paddle rotated at 75 RPM, in 900 ml of Glycine buffer pH 3.0 (0.008M) for 1 hour followed by 900 ml of phosphate buffer pH 6.8, and tested at different specified time points.

### Example 1:

### Composition:

| ***Ingredients*** | ***Weight (mg* / *tablet)*** | ***% wlw*** |
|---|---|---|
| Cephalexin monohydrate (Equiv. to 750 mg Cephalexin) | 798.15 | 75.74 |
| Lactose anhydrous | 188.15 | 18.26 |
| Xanthan gum | 21.0 | 2.0 |
| Eudragit NE 30D | 31.5 | 3.0 |
| Purified water | q.s. | - |
| Magnesium stearate | 10.5 | 1.0 |

### Dissolution profile:

| ***Time (hour)*** | ***Percent Cephalexin Released*** |
|---|---|
| 1 | 19.25 |
| 2 | 26.44 |
| 4 | 44.0 |
| 6 | 59.57 |
| 8 | 70.4 |
| 10 | 78.5 |
| 12 | 81.9 |

### Example 2:

### Composition:

| ***Ingredients*** | ***Weight (mg* / *tablet)*** | ***% w*/*w*** |
|---|---|---|
| Cephalexin monohydrate (Equiv. to 750 mg Cephalexin) | 795.32 | 75.25 |
| Lactose anhydrous | 107.68 | 10.26 |
| Xanthan gum | 31.5 | 3.0 |
| Eudragit NE 30D | 52.5 | 5.0 |
| Purified water | q.s. | - |
| HPMC E5 | 52.5 | 5.0 |
| Magnesium stearate | 10.5 | 1.0 |

### Dissolution profile:

| ***Time (hour)*** | ***Percent Cephalexin Released*** |
|---|---|
| 1 | 25.21 |
| 2 | 30.18 |
| 4 | 38.17 |
| 6 | 50.84 |
| 8 | 63.70 |
| 10 | 73.18 |
| 12 | 78.60 |
| 14 | 84.17 |

### Example 3:

### Composition:

| ***Ingredients*** | ***Weight (mg* / *tablet)*** | ***% w*/*w*** |
|---|---|---|
| Cephalexin monohydrate (Equiv. to 750 mg Cephalexin) | 795.32 | 75.24 |
| Lactose anhydrous | 97.18 | 9.26 |
| Xanthan gum | 42.0 | 4.0 |
| Eudragit NE30D | 52.5 | 5.0 |
| Purified water | q.s. | - |
| HPMC E5 | 52.5 | 5.0 |
| Magnesium stearate | 10.5 | 1.0 |

### Dissolution profile:

| ***Time (hour)*** | ***Percent Cephalexin Released*** |
|---|---|
| 1 | 22.42 |
| 2 | 30.25 |
| 4 | 41.62 |
| 6 | 48.33 |
| 8 | 54.54 |
| 10 | 60.70 |
| 12 | 66.30 |
| 14 | 71.80 |

### Example 4:

### Composition:

| ***Ingredients*** | ***Weight (mg* / *tablet)*** | ***% w*/*w*** |
|---|---|---|
| Cephalexin monohydrate (Equiv. to 750 mg Cephalexin) | 795.32 | 75.24 |
| Lactose anhydrous | 86.68 | 8.26 |
| Xanthan gum | 52.5 | 5.0 |
| Eudragit NE 30D | 52.5 | 5.0 |
| Purified water | q.s. | - |
| HPMC E5 | 52.5 | 5.0 |
| Magnesium stearate | 10.5 | 1.0 |

### Dissolution profile :

| ***Time (hour)*** | ***Percent Cephalexin Released*** |
|---|---|
| 1 | 35.57 |
| 2 | 41.96 |
| 4 | 54.46 |
| 6 | 65.00 |

### Example 5:

### Composition:

| ***Ingredients*** | ***Weight (mg* / *tablet)*** | ***% w*/*w*** |
|---|---|---|
| Cephalexin monohydrate (Equiv. to 750 mg Cephalexin) | 795.32 | 75.24 |
| Lactose anhydrous | 107.68 | 10.26 |
| Xanthan gum | 52.5 | 5.0 |
| Eudragit NE 30D | 42.0 | 4.0 |
| Purified water | q.s. | |
| HPMC E5 | 42.0 | 4.0 |
| Magnesium stearate | 10.5 | 1.0 |

### Dissolution profile:

| ***Time (hour)*** | ***Percent Cephalexin Released*** |
|---|---|
| 1 | 26.3 |
| 2 | 31.5 |
| 4 | 39.8 |
| 6 | 47.7 |
| 8 | 51.2 |
| 10 | 56.1 |
| 12 | 62.9 |
| 14 | 69.87 |

### Example 6:

### Composition:

| ***Ingredients*** | ***Weight (mg* / *tablet)*** | ***% wlw*** |
|---|---|---|
| Cephalexin monohydrate (Equiv. to 750 mg Cephalexin) | 795.32 | 75.74 |
| Lactose anhydrous | 107.68 | 9.26 |
| Xanthan gum | 42.0 | 4.0 |
| Eudragit NE 30D | 42.0 | 4.0 |
| Purified water | q.s. | - |
| HPMC E5 | 52.5 | 5.0 |
| Magnesium stearate | 10.5 | 1.0 |

### Dissolution profile:

| ***Time (hour)*** | ***Percent Cephalexin Released*** |
|---|---|
| 1 | 26.4 |
| 2 | 31.4 |
| 4 | 38.7 |
| 6 | 49.5 |
| 8 | 61.7 |
| 10 | 67.8 |
| 12 | 80.0 |
| 14 | 85.2 |

### Example 7:

### Composition:

| ***Ingredients*** | ***Weight (mg* / *tablet)*** | ***% wlw*** |
|---|---|---|
| Cephalexin monohydrate (Equiv. to 750 mg Cephalexin) | 806.02 | 76.76 |
| Lactose anhydrous | 33.98 | 3.24 |
| Xanthan gum | 73.5 | 7.0 |
| Eudragit NE 30 D | 73.5 | 7.0 |
| Purified water | q.s. | - |
| HPMC E5 | 52.5 | 5.0 |
| Magnesium Stearate | 10.5 | 1.0 |

### Dissolution Profile: USP I, 100 RPM, First two hrs in 0.1 N HCl followed by Phosphate Buffer pH 6.8 with every time point (1, 2, 4, 6, 8, 10, 12, 14, 16, 18, 20, 22 and 24 hrs) media (900 ml) change up to 24 hrs.

| ***Time (hour)*** | ***Percent Cephalexin Released*** |
|---|---|
| 1 | 25.3 |
| 2 | 32.9 |
| 4 | 39.2 |
| 6 | 41.6 |
| 8 | 44.2 |
| 10 | 46.9 |
| 12 | 49.9 |
| 14 | 53.7 |
| 16 | 56.6 |
| 18 | 59.7 |
| 20 | 63.2 |
| 22 | 66.6 |
| 24 | 69.9 |

### Example 8:

### Composition:

| ***Ingredients*** | ***Weight (mg* / *tablet)*** | ***% w*/*w*** |
|---|---|---|
| Cephalexin monohydrate (Equiv. to 750 mg Cephalexin) | 806.02 | 76.76 |
| Lactose anhydrous | 86.48 | 8.24 |
| Xanthan gum | 73.5 | 7.0 |
| Eudragit NE 30 D | 42.0 | 4.0 |
| Purified water | q.s. | - |
| HPMC5 | 31.5 | 3.0 |
| Magnesium Stearate | 10.5 | 1.0 |

### Dissolution Profile: USP I, 100 RPM, First two hrs 0.1 N HCl followed by Phosphate Buffer pH 6.8 with every time points (1, 2, 4, 6, 8, 10, 12, 14, 16,18 and 20 hrs) media (900 ml) change up to 20 hrs.

| ***Time (hour)*** | ***Percent Cephalexin Released*** |
|---|---|
| 1 | 20.4 |
| 2 | 30.2 |
| 4 | 37.0 |
| 6 | 39.0 |
| 8 | 41.7 |
| 10 | 45.6 |
| 12 | 48.6 |
| 14 | 53.3 |
| 16 | 56.7 |
| 18 | 60.4 |
| 20 | 64.0 |

### Example 9:

### Composition:

| ***Ingredients*** | ***Weight (mg* / *tablet)*** | ***% w*/*w*** |
|---|---|---|
| Cephalexin monohydrate (Equiv. to 750 mg Cephalexin) | 806.02 | 76.76 |
| Lactose anhydrous | 54.94 | 5.24 |
| Xanthan gum | 31.5 | 3.0 |
| Eudragit NE 30 D | 42.0 | 4.0 |
| Purified water | q.s. | - |
| HPMC E5 | 105.0 | 10.0 |
| Magnesium Stearate | 10.5 | 1.0 |

### Dissolution Profile: USP I, 100 RPM, First two hrs 0.1 N HCl followed by Phosphate Buffer pH 6.8 with every time point (1, 2, 4, 6, 8, 10, 12, 14, 16 and 18 hrs) media (900 ml) change up to 18 hrs.

| ***Time (hour)*** | ***Percent Cephalexin Released*** |
|---|---|
| 1 | 25.9 |
| 2 | 38.2 |
| 4 | 43.1 |
| 6 | 45.9 |
| 8 | 49.4 |
| 10 | 53.1 |
| 12 | 59.5 |
| 14 | 70.2 |
| 16 | 78.7 |
| 18 | 88.0 |

### Example 10:

### Composition:

| ***Ingredients*** | ***Weight (mg* / *tablet)*** | ***% wlw*** |
|---|---|---|
| Cephalexin monohydrate (Equiv. to 750 mg Cephalexin) | 795.15 | 75.73 |
| Lactose anhydrous | 65.85 | 6.27 |
| Xanthan gum | 73.5 | 7.0 |
| Eudragit NE 30 D | 73.5 | 7.0 |
| Purified water | q.s. | - |
| HPMC E5 | 31.5 | 3.0 |
| Magnesium Stearate | 10.5 | 1.0 |

### Dissolution Profile: USP I, 100 RPM, First two hrs 0.1 N HCl followed by Phosphate Buffer pH 6.8 with every time point (1, 2, 4, 6, 8, 10, 12, 14, 16 and 18 hrs) media (900 ml) change up to 18 hrs.

| ***Time (hour)*** | ***Percent Cephalexin Released*** |
|---|---|
| 1 | 24.3 |
| 2 | 35.7 |
| 4 | 43.1 |
| 6 | 46.2 |
| 8 | 50.9 |
| 10 | 56.8 |
| 12 | 64.2 |
| 14 | 72.1 |
| 16 | 82.2 |
| 18 | 95.7 |

### Bioavailability studies:

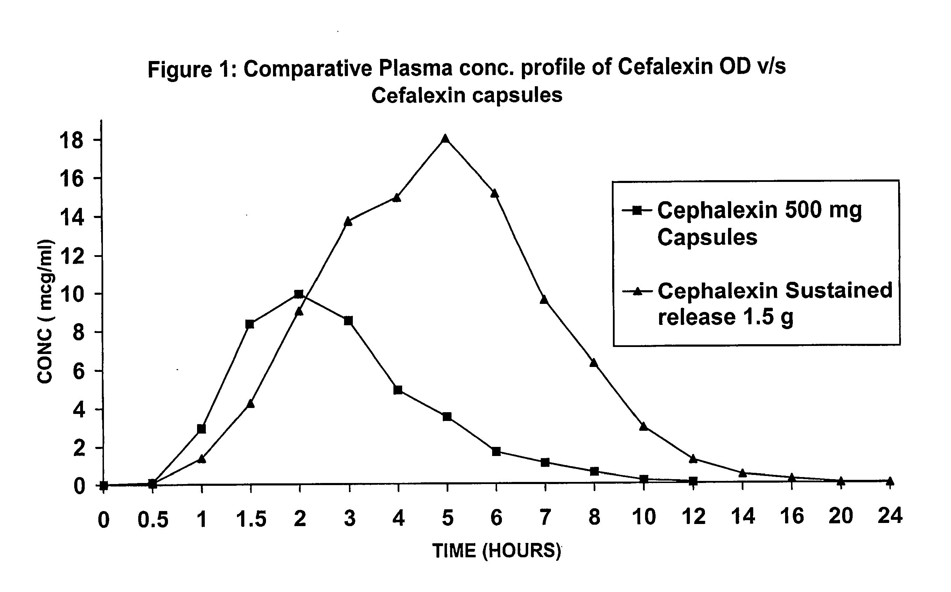

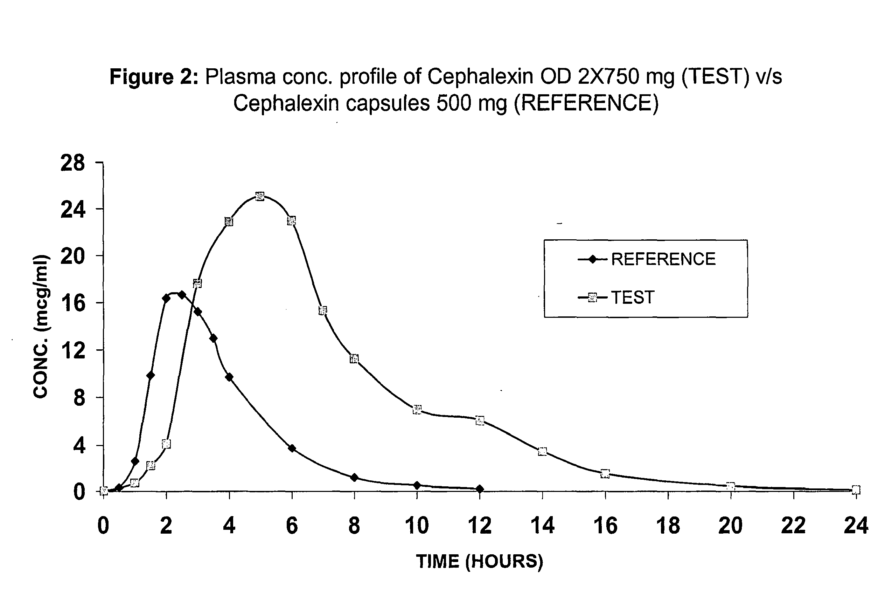

The bioavailability study was conducted for comparison between conventional Cephalexin capsule (500 mg) and each of the sustained release composition formulations of Cephalexin 2 tablets of 750mg, prepared according to the above examples of the present invention. In each of the two studies, eight healthy male volunteers were selected for the study in which each volunteer was administered a dose of the drug with 240 ml of water. The volunteers fasted overnight and had a standard breakfast before taking the drug. The desired blood levels upto 18 to 21 hours were achieved with the compositions prepared according to examples of the present invention, clearly indicating that it can be used for once daily dosing. The comparative pharmacokinetic parameters of the two studies for compositions of examples 9 & 10 are summarized in tables below(Figures 1 & 2)

The cephalosporin antibiotic exhibits minimal concentration dependent killing and produce short term or no persistent effect with most bacteria. The killing rate of these antibiotics saturates at concentrations of around 4 to 5 times the MBC, thus high concentration will not kill the bacteria faster than lower concentrations. It has also been suggested that a concentration much greater than the MIC decreases in bacterial kill potency. These findings have led to the hypothesis that continuously maintained concentrations above a certain level, related to the MIC would be more efficacious than the high peak through concentrations obtained with an intermittent dosing regimen.

From the Pharmacokinetic data obtained, it is seen the sustained release formulation has achieved the Time / MIC approximately equivalent to 3 times the dosing of conventional dosage regime which is essential for killing the bacteria.

### Comparative pharmacokinetic parameters for composition of example 9:

| **Parameters** | **Cephalexin Capsules 500mg** | **Cephalexin OD (750 mg X 2 T)** |
|---|---|---|
| Cₘₐₓ (mcg/ml) | 13.45 | 20.01 |
| Tₘₐₓ (hrs) | 2.18 | 4.87 |
| AUC₀₋ₜ (mcg.hr/ml) | 34.62 | 100.34 |

### Comparative pharmacokinetic parameters for composition of example 10:

| **Parameters** | **Cephalexin Capsules 500mg** | **Cephalexin OD (750 mg X 2 T)** |
|---|---|---|
| Cₘₐₓ (mcg/ml) | 20.10 | 26.91 |
| Tₘₐₓ (hrs) | 2.375 | 4.875 |
| AUC₀₋ₜ (mcg.hr/ml) | 61.29 | 164.27 |

### Example 11:

### Composition:

| ***Ingredients*** | ***Weight (mg* / *tablet)*** | ***% w*/*w*** |
|---|---|---|
| Cefprozil monohydrate (Equiv. to 1000 mg Cefprozil) | 1084.52 | 77.54 |
| Lactose anhydrous | 118.48 | 8.46 |
| Xanthan gum | 56.0 | 4.0 |
| Eudragit NE 30D | 56.0 | 4.0 |
| Purified water | q.s. | - |
| HPMC E5 | 70.0 | 5.0 |
| Magnesium stearate | 14.0 | 1.0 |

### Dissolution profile:

| ***Time (hour)*** | ***Percent Cefprozil Released*** |
|---|---|
| 1 | 17.4 |
| 2 | 21.2 |
| 4 | 22.0 |
| 6 | 24.2 |
| 8 | 33.1 |
| 10 | 40.2 |
| 12 | 45.6 |
| 14 | 53.0 |

### Example 12:

### Composition:

| ***Ingredients*** | ***Weight (mg* / *tablet)*** | ***% w*/*w*** |
|---|---|---|
| Cefprozil monohydrate (Equiv. to 1000 mg Cefprozil) | 1084.52 | 77.54 |
| Lactose anhydrous | 90.48 | 8.46 |
| Xanthan gum | 42.0 | 3.0 |
| Eudragit NE 30D | 28.0 | 2.0 |
| Purified water | q.s. | - |
| HPMC E5 | 140.0 | 10.0 |
| Magnesium stearate | 14.0 | 1.0 |

### Dissolution profile :

| ***Time (hour)*** | ***Percent Cefprozil Released*** |
|---|---|
| 1 | 20.3 |
| 2 | 24.4 |
| 4 | 32.1 |
| 6 | 40.8 |
| 8 | 57.4 |
| 10 | 70.5 |
| 12 | 78.5 |
| 14 | 84.8 |

### Example 13:

### Composition:

| ***Ingredients*** | ***Weight (mg* / *tablet)*** | ***% w*/*w*** |
|---|---|---|
| Cefprozil monohydrate (Equiv. to 1000 mg Cefprozil) | 1066.7 | 76.19 |
| Lactose anhydrous | 81.3 | 5.81 |
| Xanthan gum | 28.0 | 2.0 |
| Eudragit NE 30 D | 70.0 | 5.0 |
| Purified water | q.s. | - |
| HPMC E5 | 140.0 | 10.0 |
| Magnesium Stearate | 14.0 | 1.0 |

### Dissolution Profile: USP I, 100 RPM, First two hrs 0.1 N HCl followed by Phosphate Buffer pH 6.8 with every time point (1, 2, 4, 6, 8, 10, 12,14 and 16 hrs) media (900 ml) change up to 16 hrs.

| ***Time (hour)*** | ***Percent Cefprozil Released*** |
|---|---|
| 1 | 20.8 |
| 2 | 28.4 |
| 4 | 31.4 |
| 6 | 32.9 |
| 8 | 35.8 |
| 10 | 41.4 |
| 12 | 47.6 |
| 14 | 53.2 |
| 16 | 59.1 |

### Example 14:

### Composition:

| ***Ingredients*** | ***Weight (mg* / *tablet)*** | ***% w*/*w*** |
|---|---|---|
| Cefprozil monohydrate (Equiv. to 1000 mg Cefprozil) | 1066.7 | 76.19 |
| Lactose anhydrous | 95.26 | 6.81 |
| Xanthan gum | 28.0 | 2.0 |
| Eudragit NE 30 D | 56.0 | 4.0 |
| Purified water | q.s. | - |
| HPMC E5 | 140.0 | 10.0 |
| Magnesium Stearate | 14.0 | 1.0 |

### Dissolution Profile: USP I, 100 RPM, First two hrs 0.1 N HCl followed by Phosphate Buffer pH 6.8 with every time points (1, 2, 4, 6, 8, 10 and 12 hrs) media (900 ml) change up to 12 hrs.

| ***Time (hour)*** | ***Percent Cefprozil Released*** |
|---|---|
| 1 | 22.4 |
| 2 | 30.3 |
| 4 | 35.2 |
| 6 | 37.0 |
| 8 | 40.4 |
| 10 | 45.0 |
| 12 | 51.0 |

### Example 15:

### Composition:

| ***Ingredients*** | ***Weight (mg* / *tablet)*** | ***% w*/*w*** |
|---|---|---|
| Cefprozil monohydrate (Equiv. to 1000 mg Cefprozil) | 1048.3 | 74.9 |
| Lactose anhydrous | 99.67 | 7.1 |
| Xanthan gum | 42.0 | 3.0 |
| Eudragit NE 3 0 D | 56.0 | 4.0 |
| Purified water | q.s. | - |
| HPMC E5 | 140.0 | 10.0 |
| Magnesium Stearate | 14.0 | 1.0 |

### Dissolution Profile: USP I, 100 RPM, First two hrs 0.1 N HCl followed by Phosphate Buffer pH 6.8 with every time point (1, 2, 4, 6, 8, 10, 12, 14, 16, 18, 20, 22 and 24 hrs) media (900 ml) change up to 24 hrs.

| ***Time (hour)*** | ***Percent Cefprozil Released*** |
|---|---|
| 1 | 29.3 |
| 2 | 39.2 |
| 4 | 43.1 |
| 6 | 46.0 |
| 8 | 48.8 |
| 10 | 51.0 |
| 12 | 54.6 |
| 14 | 58.8 |
| 16 | 65.4 |
| 18 | 72.1 |
| 20 | 77.8 |
| 22 | 84.7 |
| 24 | 95.1 |

### Example 16:

### Composition:

| ***Ingredients*** | ***Weight (mg* / *tablet)*** | ***% w*/*w*** |
|---|---|---|
| Cefprozil monohydrate (Equiv. to 1000 mg Cefprozil) | 1048.33 | 74.9 |
| Lactose anhydrous | 127.67 | 9.1 |
| Xanthan gum | 84.0 | 6.0 |
| Eudragit NE 30 D | 56.0 | 4.0 |
| Purified water | q.s. | - |
| HPMC E5 | 70.0 | 5.0 |
| Magnesium Stearate | 14.0 | 1.0 |

### Dissolution Profile: USP I, 100 RPM, First two hrs 0.1 N HCl followed by Phosphate Buffer pH 6.8 with every time point (1, 2, 4, 6, 8, 10, 12, 14, 16, 18, 20, 22 and 24 hrs) media (900 ml) change up to 24 hrs.

| ***Time (hour)*** | ***Percent Cefprozil Released*** |
|---|---|
| 1 | 17.1 |
| 2 | 23.4 |
| 4 | 26.3 |
| 6 | 29.0 |
| 8 | 30.5 |
| 10 | 35.0 |
| 12 | 36.9 |
| 14 | 39.1 |
| 16 | 40.3 |
| 18 | 41.3 |
| 20 | 44.6 |
| 22 | 46.7 |
| 24 | 52.5 |

### Example 17:

### Composition:

| ***Ingredients*** | ***Weight (mg* / *tablet)*** | ***% w*/*w*** |
|---|---|---|
| Cefprozil monohydrate (Equiv. to 1000 mg Cefprozil) | 1048.33 | 74.9 |
| Lactose anhydrous | 57.67 | 4.1 |
| Xanthan gum | 84.0 | 6.0 |
| Eudragit NE 30 D | 56.0 | 4.0 |
| Purified water | q.s. | - |
| HPMC E5 | 140.0 | 10.0 |
| Magnesium Stearate | 14.0 | 1.0 |

### Dissolution Profile: USP I, 100 RPM, First two hrs 0.1 N HCl followed by Phosphate Buffer pH 6.8 with every time points (1, 2, 4, 6, 8, 10, 12,14,16,18,20,22 and 24 hrs) media (900 ml) change up to 24 hrs.

| ***Time (hour)*** | ***Percent Cefprozil Released*** |
|---|---|
| 1 | 15.9 |
| 2 | 23.4 |
| 4 | 27.0 |
| 6 | 29.8 |
| 8 | 31.8 |
| 10 | 38.3 |
| 12 | 44.5 |
| 14 | 46.3 |
| 16 | 47.8 |
| 18 | 49.0 |
| 20 | 52.5 |
| 22 | 55.7 |
| 24 | 60.7 |

### Example 18:

### Composition:

| ***Ingredients*** | ***Weight (mg* / *tablet)*** | ***% w*/*w*** |
|---|---|---|
| Cefprozil monohydrate (Equiv. to 1000 mg Cefprozil) | 1056.52 | 75.5 |
| Lactose anhydrous | 105.48 | 7.5 |
| Xanthan gum | 28.0 | 2.0 |
| Eudragit NE 30 D | 28.0 | 2.0 |
| Purified water | q.s. | - |
| HPMC E5 | 168.0 | 12.0 |
| Magnesium Stearate | 14.0 | 1.0 |

### Dissolution Profile: USP I, 100 RPM, First two hrs 0.1 N HCl followed by Phosphate Buffer pH 6.8 with every time point (1, 2, 4, 6, 8, 10, 12 and 14 hrs) media (900 ml) change up to 14 hrs.

| ***Time (hour)*** | ***Percent Cefprozil Released*** |
|---|---|
| 1 | 20.9 |
| 2 | 30.2 |
| 4 | 33.6 |
| 6 | 39.5 |
| 8 | 50.3 |
| 10 | 63.7 |
| 12 | 82.0 |
| 14 | 93.1 |

### Example 19:

### Composition:

| ***Ingredients*** | ***Weight (mg* / *tablet)*** | ***% w*/*w*** |
|---|---|---|
| Cefprozil monohydrate (Equiv. to 1000 mg Cefprozil) | 1070.0 | 76.43 |
| Lactose anhydrous | 35.94 | 2.57 |
| Xanthan gum | 84.0 | 6.0 |
| Eudragit NE 30 D | 56.0 | 4.0 |
| Purified water | q.s. | - |
| HPMC E5 | 140.0 | 10.0 |
| Magnesium Stearate | 14.0 | 1.0 |

### Dissolution Profile: USP I, 100 RPM, First two hrs 0.1 N HCl followed by Phosphate Buffer pH 6.8 with every time point (1, 2, 4, 6, 8, 10, 12 and 14 hrs) media (900 ml) change up to 14 hrs.

| ***Time (hour)*** | ***Percent Cefprozil Released*** |
|---|---|
| 1 | 18.9 |
| 2 | 22.8 |
| 4 | 33.3 |
| 6 | 43.3 |
| 8 | 54.7 |
| 10 | 65.0 |
| 12 | 77.2 |
| 14 | 88.0 |

The bioavailability study was conducted for comparison between conventional Cefprozil (500mg) and sustained release composition of Cefprozil (1000mg) of the above example.

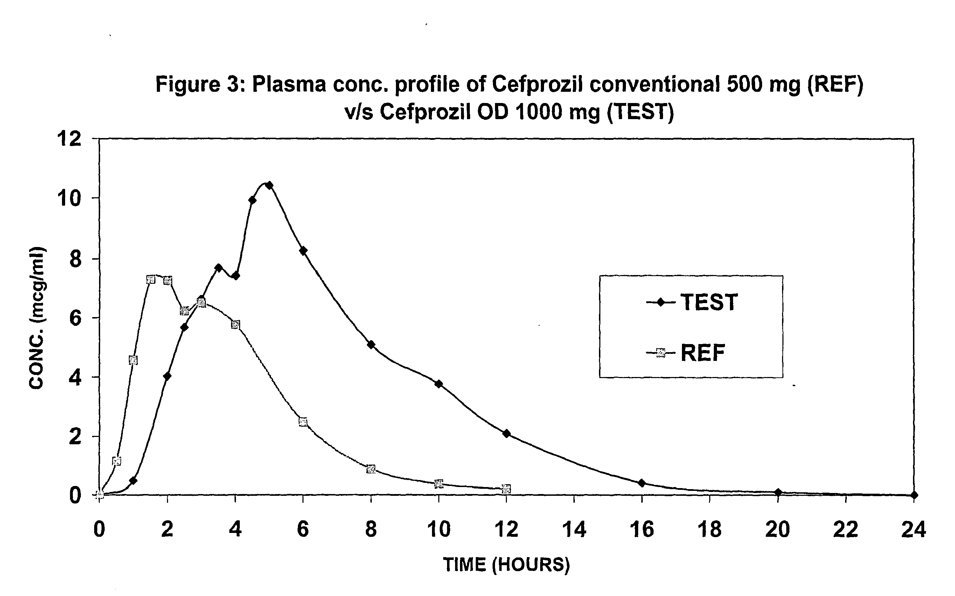

Six healthy male volunteers were selected for the study in which each volunteer was administered a dose of the drug with 180 ml of water. The volunteers fasted overnight and had a US FDA recommended breakfast before taking the drug. The desired blood levels up to 15-16 hrs were achieved with the composition prepared according the present invention, clearly indicating that it can be used as once daily dosing. The data is summarized in table below (Figure 3).

### Comparative pharmacokinetic parameters:

| **Parameters** | **Cefprozil 500 mg (Conventional)** | **Cefprozil OD 1000 mg** |
|---|---|---|
| Cₘₐₓ (mcg/ml) | 11.18 | 11.86 |
| Tₘₐₓ (hrs) | 2.66 | 5.75 |
| AUC₀₋ₜ (mcg.hr/ml) | 34.53 | 67.96 |

### Example 20:

### Composition:

| ***Ingredients*** | ***Weight (mg* / *tablet)*** | ***% w*/*w*** |
|---|---|---|
| Cefpodoxime proxetil (Equiv. to 400 mg cefpodoxime) | 563.42 | 56.34 |
| Lactose anhydrous | 371.58 | 37.16 |
| Croscarmellose Sodium | 15.0 | 1.5 |
| Calcium CMC | 20.0 | 2.0 |
| Xanthan gum | 10.0 | 1.0 |
| Eudragit NE 30 D | 10.0 | 1.0 |
| Purified water | q.s. | - |
| Magnesium Stearate | 5.0 | 0.5 |
| Croscarmellose Sodium | 25.0 | 2.5 |
| Magnesium Stearate | 10.0 | 1.0 |

### Dissolution Profile: USP II, 75 RPM, First hr in Glycine buffer pH 3.0 (0.008M) followed by Phosphate Buffer pH 6.8 with every time point (1, 2, 3, 4 and 5 hrs.) media (900 ml) change up to 5 hrs.

| ***Time (hour)*** | ***Percent Cefpodoxime proxetil Released*** |
|---|---|
| 1 | 43.9 |
| 2 | 67.6 |
| 3 | 84.2 |
| 4 | 93.5 |
| 5 | 101.8 |

The bioavailability study was conducted for comparison between conventional Cefpodoxime proxetil (200mg) bid and sustained release composition of Cefpodoxime proxetil OD (400mg) of the above example.

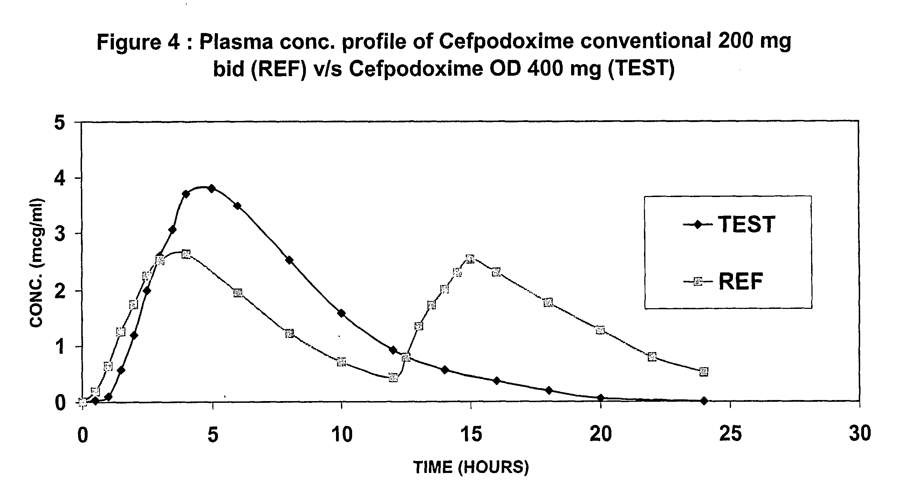

Twelve healthy male volunteers were selected for the study in which each volunteer was administered a dose of the drug with 240 ml of water. The volunteers fasted overnight and had a US FDA recommended breakfast before taking the drug. The desired blood levels up to 15-16 hrs were achieved with the composition prepared according the present invention, clearly indicating that it can be used as once daily dosing. The data is summarized in table below (Figure 4).

### Comparative pharmacokinetic parameters:

| **Parameters** | **Cefpodoxime 200 mg bid (Conventional)** | **Cefpodoxime OD 400 mg** |
|---|---|---|
| Cₘₐₓ (mcg/ml) | 3.030 | 4.223 |
| Tₘₐₓ (hrs) | 3.542 | 4.875 |
| AUC₀₋ₜ (mcg.hr/ml) | 34.839 | 29.148 |

### Example 21:

### Composition:

| ***Ingredients*** | ***Weight (mg* / *tablet)*** | ***% w*/*w*** |
|---|---|---|
| Cefuroxime axetil (Equiv. to 500 mg cefuroxime) | 614.85 | 51.23 |
| Lactose anhydrous | 153.15 | 12.76 |
| Croscarmellose Sodium | 120.0 | 10.0 |
| Calcium CMC | 36.0 | 3.0 |
| Copolyvidonum (Plasdone S-630) | 60.0 | 5.0 |
| Xanthan gum | 12.0 | 1.0 |
| Eudragit NE 30 D | 12.0 | 1.0 |
| Purified water | q.s. | - |
| Hydrogenated vegetable oil | 6.0 | 0.5 |
| Calcium CMC | 60.0 | 5.0 |
| Croscarmellose Sodium | 120.0 | 10.0 |
| Hydrogenated vegetable oil | 6.0 | 0.5 |

### Dissolution Profile: USP II, 75 RPM, First hr in Glycine buffer pH 3.0 (0.008M) followed by Phosphate Buffer pH 6.8 with every time point (1, 2, 3, 4 and 5 hrs.) media (900 ml) change up to 5 hrs.

| ***Time (hour)*** | ***Percent Cefuroxime axetil Released*** |
|---|---|
| 1 | 31.3 |
| 2 | 56.7 |
| 3 | 78.9 |
| 4 | 91.7 |
| 5 | 96.5 |

### Example 22:

### Composition:

| ***Ingredients*** | ***Weight (mg* / *tablet)*** | ***% w*/*w*** |
|---|---|---|
| Cefuroxime axetil (Equiv. to 500 mg cefuroxime) | 614.85 | 51.24 |
| Lactose anhydrous | 213.15 | 17.76 |
| Croscarmellose Sodium | 120.0 | 10.0 |
| Calcium CMC | 36.0 | 3.0 |
| Copolyvidonum (Plasdone S-630) | 60.0 | 5.0 |
| Xanthan gum | 12.0 | 1.0 |
| Eudragit NE 30 D | 12.0 | 1.0 |
| Purified water | q.s | - |
| Hydrogenated vegetable oil | 6.0 | 0.5 |
| Croscarmellose Sodium | 120.0 | 10.0 |
| Hydrogenated vegetable oil | 6.0 | 0.5 |

### Dissolution Profile: USP II, 75 RPM, First hr in Glycine buffer pH 3.0 (0.008M) followed by Phosphate Buffer pH 6.8 with every time point (1, 2, 3, 4 and 5 hrs.) media (900 ml) change up to 5 hrs.

| ***Time (hour)*** | ***Percent Cefuroxime axetil Released*** |
|---|---|
| 1 | 39.3 |
| 2 | 53.1 |
| 3 | 67.4 |
| 4 | 78.4 |
| 5 | 87.4 |

### Example 23:

### Composition:

| ***Ingredients*** | ***Weight (mg* / *tablet)*** | ***% w*/*w*** |
|---|---|---|
| Cefuroxime axetil (Equiv. to 500 mg cefuroxime) | 614.85 | 51.24 |
| Lactose anhydrous | 153.15 | 12.76 |
| Croscarmellose Sodium | 120.0 | 10.0 |
| Calcium CMC | 36.0 | 3.0 |
| Copolyvidonum (Plasdone S-630) | 60.0 | 5.0 |
| Xanthan gum | 12.0 | 1.0 |
| Eudragit NE 30 D | 12.0 | 1.0 |
| Purified water | q.s | - |
| Hydrogenated vegetable oil | 6.0 | 0.5 |
| Calcium CMC | 60.0 | 5.0 |
| Croscarmellose Sodium | 120.0 | 10.0 |
| Hydrogenated vegetable oil | 6.0 | 0.5 |

### Dissolution Profile: USP II, 75 RPM, First hr in Glycine buffer pH 3.0 (0.008M) followed by Phosphate Buffer pH 6.8 with every time point (1, 2, 3, 4 and 5 hrs.) media (900 ml) change up to 5 hrs.

| ***Time (hour)*** | ***Percent Cefuroxime axetil Released*** |
|---|---|
| 1 | 31.3 |
| 2 | 56.7 |
| 3 | 78.9 |
| 4 | 91.7 |
| 5 | 96.5 |

### Example 24:

### Composition:

| ***Ingredients*** | ***Weight (mg* / *tablet)*** | ***% w*/*w*** |
|---|---|---|
| Cefadroxil monohydrate (Equiv. to 1000 mg Cefadroxil) | 1061.96 | 75.9 |
| Lactose anhydrous | 86.04 | 6.1 |
| Xanthan gum | 28.0 | 2.0 |
| Eudragit NE 30 D | 70.0 | 5.0 |
| Purified water | q.s. | - |
| HPMC E5 | 140.0 | 10.0 |
| Magnesium Stearate | 14.0 | 1.0 |

### Dissolution Profile: USP I, 100 RPM, First two hrs 0.1 N HCl followed by Phosphate Buffer pH 6.8 with every time point (1, 2, 4, 6, 8,10 and 12 hrs) media (900 ml) change up to 12 hrs.

| ***Time (hour)*** | ***Percent Cefadroxil Released*** |
|---|---|
| 1 | 26.2 |
| 2 | 36.6 |
| 4 | 41.6 |
| 6 | 44.0 |
| 8 | 46.6 |
| 10 | 50.3 |
| 12 | 55.5 |

### Example 25:

### Composition:

| ***Ingredients*** | ***Weight (mg* / *tablet)*** | ***% w*/*w*** |
|---|---|---|
| Cefadroxil monohydrate | 1061.96 | 75.9 |
| (Equiv. to 1000 mg Cefadroxil) | | |
| Lactose anhydrous | 100.04 | 7.1 |
| Xanthan gum | 28.0 | 2.0 |
| Eudragit NE 30 D | 56.0 | 4.0 |
| Purified water | q.s. | - |
| HPMC E5 | 140.0 | 10.0 |
| Magnesium Stearate | 14.0 | 1.0 |

### Dissolution Profile: USP I, 100 RPM, First two hrs 0.1 N HCl followed by Phosphate Buffer pH 6.8 with every time point (1, 2, 4, 6, 8, 10 and 12 hrs) media (900 ml) change up to 12 hrs.

| ***Time (hour)*** | ***Percent Cefadroxil Released*** |
|---|---|
| 1 | 25.5 |
| 2 | 37.4 |
| 4 | 42.1 |
| 6 | 44.2 |
| 8 | 46.7 |
| 10 | 49.4 |

### Example 26:

### Composition:

| ***Ingredients*** | ***Weight (mg* / *tablet)*** | ***% w*/*w*** |
|---|---|---|
| Cefadroxil monohydrate (Equiv. to 1000 mg Cefadroxil) | 1061.96 | 75.9 |
| Lactose anhydrous | 100.04 | 7.1 |
| Xanthan gum | 28.0 | 2.0 |
| Eudragit NE 30 D | 28.0 | 2.0 |
| Purified water | q.s. | - |
| HPMC E5 | 168.0 | 12.0 |
| Magnesium Stearate | 14.0 | 1.0 |

### Dissolution Profile: USP I, 100 RPM, First two hrs 0.1 N HCl followed by Phosphate Buffer pH 6.8 with every time point (1, 2, 4, 6, 8, 10, 12, 14 and 16 hrs) media (900 ml) change up to 16 hrs.

| ***Time (hour)*** | ***Percent Cefadroxil Released*** |
|---|---|
| 1 | 26.6 |
| 2 | 38.5 |
| 4 | 44.4 |
| 6 | 46.5 |
| 8 | 54.1 |
| 10 | 64.6 |
| 12 | 74.7 |
| 14 | 84.1 |
| 16 | 93.6 |

### Example 27:

### Composition:

| ***Ingredients*** | ***Weight (mg* / *tablet)*** | ***% w*/*w*** |
|---|---|---|
| Cefadroxil monohydrate (Equiv. to 1000 mg Cefadroxil) | 1065.21 | 76.09 |
| Lactose anhydrous | 124.79 | 8.91 |
| Xanthan gum | 28.0 | 2.0 |
| Eudragit NE 30 D | 28.0 | 2.0 |
| Purified water | q.s. | - |
| HPMC E5 | 140.0 | 10.0 |
| Magnesium Stearate | 14.0 | 1.0 |

### Dissolution Profile: USP I, 100 RPM, First two hrs 0.1 N HCl followed by Phosphate Buffer pH 6.8 with every time point (1, 2, 3, 4, 5, 6, 7 and 8 hrs) media (900 ml) change up to 8 hrs.

| ***Time (hour)*** | ***Percent Cefadroxil Released*** |
|---|---|
| 1 | 26.2 |
| 2 | 40.0 |
| 3 | 47.1 |
| 4 | 53.6 |
| 5 | 63.4 |
| 6 | 74.5 |
| 7 | 81.5 |
| 8 | 90.4 |

The bioavailability study was conducted for comparison between conventional Cefadroxil (1000mg) and sustained release composition of Cefadroxil OD 1000 mg of the above example.

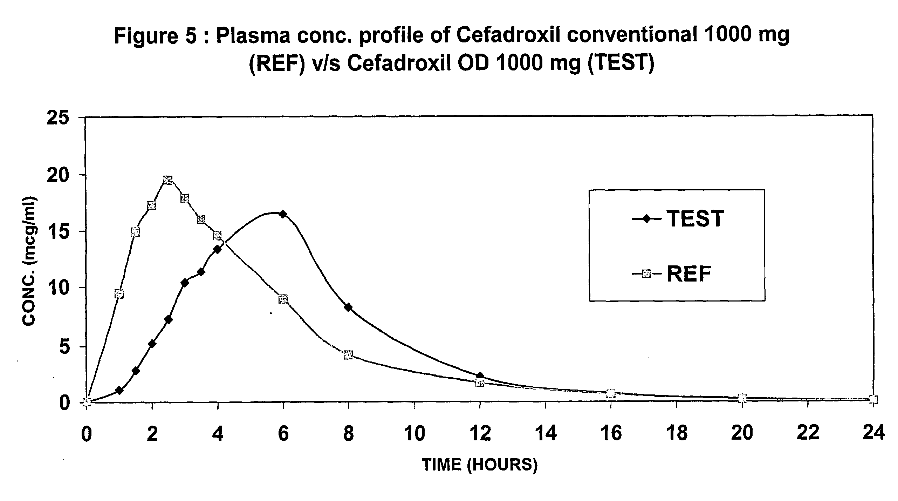

Eight healthy male volunteers were selected for the study in which each volunteer was administered a dose of the drug with 240 ml of water. The volunteers fasted overnight and had a US FDA recommended breakfast before taking the drug. The desired blood levels up to 8-10 hrs were achieved with the composition prepared according the present invention, clearly indicating that it can be used as once daily dosing. The data is summarized in table below (Figure 5).

### Comparative pharmacokinetic parameters:

| **Parameters** | **Cefadroxil 1000 mg (Conventional)** | **Cefadroxil OD 1000 mg** |
|---|---|---|
| Cₘₐₓ (mcg/ml) | 24.69 | 19.30 |
| Tₘₐₓ (hrs) | 3.31 | 4.76 |
| AUC₀₋ₜ (mcg.hr/ml) | 106.0 | 107.14 |

### List of Figures :

Figures 1 & 2 : Plot of comparative plasma profile of Cephalexin OD *v*/*s* Cephalexin capsules, for examples 9 & 10 respectively.
Figure 3 : Plot of comparative plasma profile of Cefprozil OD 1000 mg v/s Cefprozil 500 mg conventional tablets.
Figure 4 : Plot of comparative plasma profile of Cefpodoxime OD 400 mg v/s Cefpodoxime 200 mg bid conventional tablets.
Figure 5 : Plot of comparative plasma profile of Cefadroxil OD 1000 mg v/s Cefadroxil 1000 mg conventional tablets.

## Claims

1. A sustained release pharmaceutical composition comprising a cephalosporin antibiotic, 1% to 3% by weight of a mixture of polymers comprising a galactomannan being xanthan gum and a neutral swellable polymer being poly (ethyl acrylate: methyl methacrylate) 2:1 and other pharmaceutically acceptable excipients; wherein the composition comprises from 30% to 90% by weight of a cephalosporin antibiotic, from 0.1 % to 15 % by weight of xanthan gum and from 0.1 % to 15 % by weight of poly (ethyl acrylate: methyl methacrylate) 2:1.

2. The composition according to claim 1, which comprises 30% to 90% by weight of cephalosporin antibiotic, 1% to 20% by weight of mixture of said polymers comprising of galactomannans in an amount from 0.1 % to 12% by weight and neutral swellable polymer in an amount from 0.1% to 12% by weight of sustained release composition.

3. The composition according to claim 1 comprising 1% to 7% by weight of xanthan gum and 1% to 7% by weight of poly (ethyl acrylate:methyl methacrylate) 2:1.

4. The composition as claimed in claim 1, in the form of a tablet.

5. The composition as claimed in claim 1, wherein the cephalosporin antibiotic is released at a rate suitable for once daily or twice daily administration.

6. The composition according to claim 1, wherein the cephalosporin antibiotic is selected from cephalexin, cefprozil, cefditoren pivoxil, cefadroxil, cefpodoxime proxetil, cefuroxime axetil, cefaclor, cefamandole, cefoxitin, cephalothin, cephapirin, ceftizoxime, cefonicid and their pharmaceutically acceptable hydrates, salts and esters.

7. The composition according to claim 1, wherein the excipients are water soluble or water dispersible diluents, disintegrants, binders, lubricants, plasticizers, film forming agents, etc., used either alone or in combination thereof.

8. The composition according to claim 7, wherein the water soluble or water dispersible diluent comprises 1 to 30 % by weight of the composition.

9. The composition as claimed in claim 7, wherein the water soluble diluents are lactose, mannitol, glucose, sorbitol, maltose, dextrates or dextrins.

10. The composition as claimed in claim 7, wherein the water dispersible diluent is microcrystalline cellulose, pregelatinized starch, croscarmellose sodium, sodium starch glycollate, calcium carboxymethyl cellulose, copolyvidonum (Plasdone S-630), or mixtures thereof.

11. The composition as claimed in claim 7, wherein the tablet binder concentration is in the range of 0.2 % to 12 % by weight of the total weight of composition.

12. The composition as claimed in claim 7, wherein the binder is selected from polyvinyl pyrrolidone, hydroxypropyl methylcellulose, hydroxypropyl cellulose, hydroxyethyl cellulose , gelatin, pregelatinized starch, or sugar.

13. The composition as claimed in claim 7, wherein the lubricant concentration is in the range of 0.2 % to 5 % by weight of the total weight of composition.

14. The composition as claimed in claim 7, wherein the lubricant used is selected from talc, stearic acid, magnesium stearate, colloidal silicon dioxide, calcium stearate, zinc stearate, hydrogenated vegetable oil, or mixtures thereof.

15. A process for the preparation of the sustained release pharmaceutical composition as claimed in claim 1, the said method comprising the steps of:
(i) mixing the active ingredient, excipients and xanthan gum in a mixer,
(ii) granulating the mixture with poly (ethyl acrylate: methyl methacrylate) 2:1.
(iii) drying the granules by either tray drier or fluid bed drier,
(iv) milling the dried granules followed by addition and blending of dry binder and lubricant(s),
(v) compressing the lubricated granules into tablets using a tablet press and, if desired, coating the tablets.

16. A process for the preparation of the sustained release pharmaceutical composition as claimed in claim 1, the said method comprising the steps of:
(i) mixing the active ingredient, excipients and xanthan gum in a mixer;
(ii) compacting the mixture and sizing it by passing through the sieve;
(iii) granulating the mixture with poly (ethyl acrylate: methyl methacrylate) 2:1;
(iv) drying the granules by either tray drier or fluid bed drier;
(v) milling the dried granules followed by addition and blending of dry binder and lubricant(s);
(vi) compressing the lubricated granules into tablets using a tablet press and, if desired, coating the tablets.

## Patentansprüche

1. Pharmazeutische Zusammensetzung mit anhaltender Freisetzung umfassend ein Cephalosporin-Antibiotikum, 1 bis 30 Gewichts-% einer Mischung von Polymeren umfassend ein Galaktomannan, das Xanthan ist, und ein neutrales quellfähiges Polymer, das Polyethylacrylat: Methylmethacrylat 2:1 ist, und andere pharmazeutisch geeignete Trägerstoffe; wobei die Zusammensetzung von 30 bis 90 Gewichts-% eines Cephalosporin-Antibiotikums, von 0,1 bis 15 Gewichts-% Xanthan und von 0,1 bis 15 Gewichts-% Polyethylacrylat: Methylmethacrylat 2:1 umfasst.

2. Zusammensetzung nach Anspruch 1, die 30 bis 90 Gewichts-% eines Cephalosporin-Antibiotikums, 1 bis 20 % Gewichts-% einer Mischung dieser Polymere umfassend Galactomannane in einer Menge von 0,1 bis 12 Gewichts-% und neutralem quellfähigen Polymer in einer Menge von 0,1 bis 12 Gewichts-% der Zusammensetzung mit anhaltender Freisetzung umfasst.

3. Zusammensetzung nach Anspruch 1 umfassend 1 bis 7 Gewichts-% Xanthan und 1 bis 7 Gewichts-% Polyethylacrylat: Methylmethacrylat 2:1.

4. Zusammensetzung wie in Anspruch 1 beansprucht, in Form einer Tablette.

5. Zusammensetzung wie in Anspruch 1 beansprucht, wobei das Cephalosporin-Antibiotikum mit einer Geschwindigkeit freigesetzt wird, die geeignet für eine Verabreichung einmal täglich oder zweimal täglich ist.

6. Zusammensetzung nach Anspruch 1, wobei das Cephalosporin-Antibiotikum ausgewählt ist aus Cephalexin, Cefprozil, Cefditoren-Pivoxil, Cefadroxil, Cefpodoxim-Proxetil, Cefuroxim-Axetil, Cefaclor, Cefamandol, Cefoxitin, Cephalothin, Cephapirin, Ceftizoxim, Cefonicid und ihre pharmazeutisch geeigneten Hydrate, Salze und Ester.

7. Zusammensetzung nach Anspruch 1, wobei die Trägerstoffe wasserlösliche oder in Wasser dispergierbare Verdünnungsmittel, Abbaumittel, Bindemittel, Gleitmittel, Weichmacher, filmbildende Mittel, etc. sind, die entweder alleine oder in einer Kombination davon verwendet werden.

8. Zusammensetzung nach Anspruch 7, wobei das wasserlösliche oder in Wasser dispergierbare Verdünnungsmittel 1 bis 30 Gewichts-% der Zusammensetzung umfasst.

9. Zusammensetzung wie in Anspruch 7 beansprucht, wobei die wasserlöslichen Verdünnungsmittel Laktose, Mannitol, Glucose, Sorbitol, Maltose, Dextrate oder Dextrine sind.

10. Zusammensetzung wie in Anspruch 7 beansprucht, wobei das in Wasser dispergierbare Verdünnungsmittel mikrokristalline Cellulose, vorgelatinierte Stärke, Croscarmellose-Natrium, Natrium-Stärke-Glycolat, Calciumcarboxymethylcellulose, Copolyvidon (Plasdon S-630), oder Mischungen davon ist.

11. Zusammensetzung wie in Anspruch 7 beansprucht, wobei die Konzentration an Tablettenbindemittel im Bereich von 0,2 bis 12 % Gewichts-% des gesamten Gewichts der Zusammensetzung ist.

12. Zusammensetzung wie in Anspruch 7 beansprucht, wobei das Bindemittel ausgewählt ist aus Polyvinylpyrrolidon, Hydroxypropyl-Methylcellulose, Hydroxypropylcellulose, Hydroxyethylcellulose, Gelatine, vorgelatinierter Stärke oder Zucker.

13. Zusammensetzung wie in Anspruch 7 beansprucht, wobei die Konzentration des Gleitmittels im Bereich von 0,2 bis 5 Gewichts-% des Gesamtgewichts der Zusammensetzung ist.

14. Zusammensetzung wie in Anspruch 7 beansprucht, wobei das verwendete Gleitmittel ausgewählt ist aus Talkum, Stearinsäure, Magnesiumstearat, kolloidalem Siliciumdioxid, Calciumstearat, Zinkstearat, hydriertem pflanzlichen Öl, oder Mischungen davon.

15. Verfahren zur Herstellung der pharmazeutischen Zusammensetzung mit anhaltender Freisetzung wie in Anspruch 1 beansprucht, wobei das Verfahren die Schritte umfasst:
(i) Mischen des wirksamen Bestandteils, der Trägerstoffe und Xanthan in einem Mischer,
(ii) Granulieren der Mischung mit Polyethylacrylat: Methylmethacrylat 2:1,
(iii) Trocknen der Körner entweder durch einen Plattentrockner oder Wirbelschichttrockner,
(iv) Mahlen der getrockneten Körner gefolgt durch die Zugabe und dem Vermischen von trockenem Bindemittel und Gleitmittel(n),
(v) Pressen der mit Gleitmittel versehenen Körner in Tabletten unter Verwendung einer Tablettenpresse und, wenn gewünscht, Umhüllen der Tabletten.

16. Verfahren zur Herstellung der pharmazeutischen Zusammensetzung mit anhaltender Freisetzung, wie in Anspruch 1 beansprucht, wobei das Verfahren die Schritte umfasst:
(i) Mischen des aktiven Bestandteils, der Trägerstoffe und Xanthan in einem Mischer,
(ii) Verdichten der Mischung und ihr Sortieren nach Größe durch Durchführen durch das Sieb;
(iii) Granulieren der Mischung mit Polyethylacrylat: Methylmethacrylat 2:1,
(iv) Trocknen der Körner entweder durch einen Plattentrockner oder Wirbelschichttrockner,
(v) Mahlen der getrockneten Körner gefolgt durch die Zugabe und dem Vermischen von trockenem Bindemittel und Gleitmittel(n),
(vi) Pressen der mit Gleitmittel versehenen Körner in Tabletten unter Verwendung einer Tablettenpresse und, wenn gewünscht, Umhüllen der Tabletten.

## Revendications

1. Composition pharmaceutique à libération prolongée comprenant un antibiotique de la famille des céphalosporines, 1 % à 30 % en poids d'un mélange de polymères comprenant un galactomannane étant de la gomme xanthane et un polymère gonflable neutre étant le poly(acrylate d'éthyle-méthacrylate de méthyle) 2:1, et d'autres excipients pharmaceutiquement acceptables, la composition comprenant de 30 % à 90 % en poids d'antibiotique de la famille des céphalosporines, de 0,1 % à 15 % en poids de gomme xanthane, et de 0,1 % à 15 % en poids de poly(acrylate d'éthyle-méthacrylate de méthyle) 2:1.

2. Composition selon la revendication 1, qui comprend 30 % à 90 % en poids d'antibiotique de la famille des céphalosporines, 1 % à 20 % en poids d'un mélange desdits polymères comprenant des galactomannanes dans une quantité de 0,1 % à 12 % en poids et un polymère gonflable neutre dans une quantité de 0,1 % à 12 % en poids de composition à libération prolongée.

3. Composition selon la revendication 1 comprenant 1 % à 7 % en poids de gomme xanthane et 1 % à 7 % en poids de poly(acrylate d'éthyle-méthacrylate de méthyle) 2:1.

4. Composition selon la revendication 1, sous la forme d'un comprimé.

5. Composition selon la revendication 1, dans laquelle l'antibiotique de la famille des céphalosporines est libéré à une vitesse appropriée pour administration une fois par jour ou deux fois par jour.

6. Composition selon la revendication 1, dans laquelle l'antibiotique de la famille des céphalosporines est choisi parmi la céphalexine, le cefprozil, le cefditoren pivoxil, le céfadroxil, le cefpodoxime proxétil, le céfuroxime axétil, le céfaclor, la céfamandole, la céfoxitine, la céphalothine, la céphapirine, le ceftizoxime, le céfonicide et leurs hydrates, sels ou esters pharmaceutiquement acceptables.

7. Composition selon la revendication 1, dans laquelle les excipients sont des diluants, des délitants, des liants, des lubrifiants, des plastifiants, des agents filmogènes, etc., solubles dans l'eau ou dispersibles dans l'eau, utilisés seuls ou dans une combinaison de ceux-ci.

8. Composition selon la revendication 7, dans laquelle le diluant soluble dans l'eau ou dispersible dans l'eau représente 1 à 30 % en poids de la composition.

9. Composition selon la revendication 7, dans laquelle le diluant soluble dans l'eau est le lactose, le mannitol, le glucose, le sorbitol, le maltose, un dextrate ou une dextrine.

10. Composition selon la revendication 7, dans laquelle le diluant dispersible dans l'eau est la cellulose microcristalline, l'amidon prégélatinisé, la croscarmellose sodique, le glycolate d'amidon sodique, la carboxyméthylcellulose calcique, la copolyvidone (Plasdone S-630), ou un mélange de ceux-ci.

11. Composition selon la revendication 7, dans laquelle la concentration de liant dans le comprimé se situe dans la gamme de 0,2 % à 12 % en poids, rapporté au poids total de composition.

12. Composition selon la revendication 7, dans laquelle le liant est choisi parmi la polyvinylpyrrolidone, l'hydroxypropylméthylcellulose, l'hydroxypropylcellulose, l'hydroxyéthylcellulose, la gélatine, l'amidon prégélatinisé et le sucre.

13. Composition selon la revendication 7, dans laquelle la concentration de lubrifiant se situe dans la gamme de 0,2 % à 5 % en poids, rapporté au poids total de composition.

14. Composition selon la revendication 7, dans laquelle le lubrifiant utilisé est choisi parmi le talc, l'acide stéarique, le stéarate de magnésium, le dioxyde de silicium colloïdal, le stéarate de calcium, le stéarate de zinc, l'huile végétale hydrogénée, et les mélanges de ceux-ci.

15. Procédé de préparation de la composition pharmaceutique à libération prolongée selon la revendication 1, ledit procédé comprenant les étapes de :
(i) mélange de l'ingrédient actif, des excipients et de la gomme xanthane dans un malaxeur,
(ii) granulation du mélange avec du poly(acrylate d'éthyle-méthacrylate de méthyle) 2:1,
(iii) séchage des granulés avec un séchoir à plateaux ou un séchoir à lit fluide,
(iv) broyage des granulés séchés puis addition et mélange d'un liant sec et d'un/de lubrifiant(s),
(v) compression des granulés lubrifiés en comprimés à l'aide d'une presse à comprimés et, si on le souhaite, enrobage des comprimés.

16. Procédé de préparation de la composition pharmaceutique à libération prolongée selon la revendication 1, ledit procédé comprenant les étapes de :
(i) mélange de l'ingrédient actif, des excipients et de la gomme xanthane dans un malaxeur,
(ii) compaction du mélange et dimensionnement de celui-ci par passage à travers un tamis,
(iii) granulation du mélange avec du poly(acrylate d'éthyle-méthacrylate de méthyle) 2:1,
(iv) séchage des granulés avec un séchoir à plateaux ou un séchoir à lit fluide,
(v) broyage des granulés séchés puis addition et mélange d'un liant sec et d'un/de lubrifiant(s),
(vi) compression des granulés lubrifiés en comprimés à l'aide d'une presse à comprimés et, si on le souhaite, enrobage des comprimés.
